(19) Europäisches Patentamt
European Patent Office
Office européen des brevets

(11) Veröffentlichungsnummer: **0 127 836**
**B1**

(12) # EUROPÄISCHE PATENTSCHRIFT

(45) Veröffentlichungstag der Patentschrift: **13.08.86**

(51) Int. Cl.⁴: **C 07 C 53/02**, C 07 C 51/44

(21) Anmeldenummer: **84105831.6**

(22) Anmeldetag: **22.05.84**

(54) **Verfahren zur destillativen Gewinnung von Ameisensäure.**

(30) Priorität: **31.05.83 DE 3319651**

(43) Veröffentlichungstag der Anmeldung: **12.12.84 Patentblatt 84/50**

(45) Bekanntmachung des Hinweises auf die Patenterteilung: **13.08.86 Patentblatt 86/33**

(84) Benannte Vertragsstaaten: **AT BE CH DE FR GB IT LI NL SE**

(56) Entgegenhaltungen: **EP-A-0 017 866**

(73) Patentinhaber: **BASF Aktiengesellschaft, Carl-Bosch- Strasse 38, D-6700 Ludwigshafen (DE)**

(72) Erfinder: **Bott, Kaspar, Dr., Rieslingweg 4, D-6706 Wachenheim (DE)**
Erfinder: **Kaibel, Gerd, Robert- Bosch- Strasse 4, D-6840 Lampertheim (DE)**
Erfinder: **Hartmann, Horst, Lindenstrasse 45, D-6737 Boehl- Iggelheim (DE)**
Erfinder: **Irnich, Rudolf, Dr., In den Hahndornen 2, D-6719 Bobenheim (DE)**
Erfinder: **Buelow, Horst, Dr., Max- Slevogt- Strasse 12d, D-6710 Frankenthal (DE)**

**EP 0 127 836 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft ein verbessertes Verfahren zur destillativen Gewinnung von Ameisensäure aus deren Gemischen mit Lösungsmitteln der allgemeinen Formel I

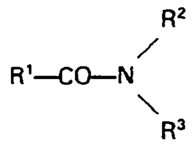

I

in der $R^1$ für Wasserstoff, die Methyl-, Ethyl- oder Vinylgruppe steht, und in der $R^2$ und $R^3$ Alkyl-, Cycloalkyl-, Aryl- oder Aralkylgruppen oder gemeinsam eine 1,4- oder 1,5-Alklengruppe mit jeweils 1 bis 8 C-Atomen mit der Maßgabe bedeuten, daß die Summe der C-Atome von $R^2$ und $R^3$ 7 bis 14 beträgt und daß nur einer dieser Reste eine Arylgruppe ist.

Derartige Gemische fallen in der Technik an, wenn man die Ameisensäure aus ihren wäßrigen Lösungen mit den Lösungsmitteln I extrahiert, um auf diese Weise sowie durch anschließende Destillation der Extraktphase zur wasserfreien oder weitgehend wasserfreien Säure zu gelangen (vgl. DE—A 25 45 658). Für die großtechnische Herstellung von wasserfreier oder weitgehend wasserfreier Ameisensäure ist die Verwendung dieser Verbindungen, darunter vor allem des N-Di-n-butylformamids, ferner in den EP—A 00 12 321 und 00 17 866 beschrieben.

Allen diesen Verfahren ist gemeinsam, daß man ein Gemisch aus dem Lösungsmittel I und Ameisensäure sowie gegebenenfalls noch etwas Wasser erhält, von welchem die Säure bzw. die wasserhaltige Säure abdestilliert werden muß.

Da die Extraktionswirkung der Lösungsmittel I, sei es bei der flüssig-flüssig-Extraktion oder der Extraktivdestillation, aber gerade darauf beruht, daß die Säure je nach Basenstärke von I mehr oder weniger stark salzartig an I gebunden wird, erfordert die Spaltung dieser Bindungen über die reine Verdampfungsenergie der Säure hinaus zusätzliche Energie und damit eine zusätzliche thermische Belastung des Destillationsgutes. Auch wenn man die Destillation unter vermindertem Druck vornimmt, wobei ein Druck von 40—60 mbar abs. aus wirtschaftlichen Gründen nicht unterschritten werden soll, damit die Raum-Zeit-Ausbeute nicht zu gering wird und damit zur Kondensation des Destillates keine Kälte erzeugt werden muß, liegen die Sumpftemperaturen immer noch zwischen etwa 150 und 170°C, um die Säure vollständig von I abzutrennen. Bei diesen Temperaturen finden aber bereits diverse unerwünschte Reaktionen statt, und zwar vor allem Zersetzung der Ameisensäure.

Der Erfindung lag daher die Aufgabe zugrunde, die Destillation der definitionsgemäßen Gemische so zu gestalten, daß deren thermische Belastung vermindert wird, ohne daß die Wirtschaftlichkeit der Destillation darunter leidet.

Demgemäß wurde gefunden, daß man Ameisensäure aus ihren Gemischen mit Lösungsmitteln der allgemeinen Formel I

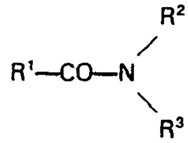

I

in der $R^1$ für Wasserstoff, die Methyl-, Ethyl- oder Vinylgruppe steht, und in der $R^2$ und $R^3$ Alkyl-, Cycloalkyl-, Aryl- oder Aralkylgruppen oder gemeinsam eine 1,4- oder 1,5-Alkylengruppe mit jeweils 1 bis 8 C-Atomen mit der Maßgabe bedeuten, daß die Summe der C-Atome von $R^2$ und $R^3$ 7 bis 14 beträgt und daß nur einer dieser Reste eine Arylgruppe ist, auf wirtschaftliche und technische vorteilhafte Weise destillativ gewinnen kann, wenn man die Destillation in Gegenwart eines Carbonsäureamids II vornimmt, das tiefer als das Lösungsmittel I siedet.

Als besonders vorteilhaft haben sich Formamide erwiesen, und zwar vor allem solche, die am Stickstoffatom einfach oder doppelt mit $C_1$- bis $C_4$-Alkylgruppen, vor allem der Methylgruppe, substituiert sind. Weiterhin kommen die entsprechenden Acetamide und Propionamide in Betracht, sofern sie in dem jeweiligen System die Siedepunktsbedingung erfüllen.

Beispiele für geeignete Paare I und II sind der nachstehenden Tabelle zu entnehmen, wobei die Siedepunkte unter Normaldruck angegeben wurden. Für das Arbeiten unter vermindertem Druck bleiben die Relationen im wesentlichen gewahrt.

TABELLE

Geeignete Paare des Lösungsmittels I und des Carbonsäureamides II zur destillativen Gewinnung von Ameisensäure (Sdp. 101°C).

| Lösungsmittel I Art | Sdp. °C | Carbonsäureamid II Art | Sdp. °C |
|---|---|---|---|
| Di-n-butylformamid | 247,1 | Formamid | 212,1 |
| | | Methylformamid | 199,3 |
| | | Dimethylformamid | 153,0 |
| | | Acetamid | 222,0 |
| | | N,N-Dimethylacetamid | 165,0 |
| Di-n-pentylformamid | | Formamid | 212,1 |
| | ca. 280 | Methylformamid | 199,3 |
| | | Dimethylformamid | 153,0 |
| | | Acetamid | 222,0 |

Das erfindungsgemäße Verfahren hat die größte Bedeutung bei der extraktiven Gewinnung der Ameisensäure aus solchen wäßrigen Lösungen mittels der Lösungsmittel I als Extraktionsmittel, wie in sie bei der Hydrolyse von Methylformiat anfallen. Es ist jedoch hierauf nicht beschränkt und kann daher auch auf die Trennung von entsprechenden Gemischen anderer Provenienz angewandt werden.

Allgemein gilt als Regel, daß das Carbonsäureamid II bei Normaldruck etwa 20—80°C tiefer sieden soll als das Lösungsmittel I und etwa 50—120°C höher als die Ameisensäure, wobei es besonders vorteilhaft ist, wenn der Siedpunkt von II ungefähr in der Mitte des Intervalls liegt, welches durch die Siedepunkte von I und der Ameisensäure gegeben ist. Ferner ist es zweckmäßig, daß die Siedepunkte von I und der Ameisensäure um mindestens 50°C, vorzugsweise um 100—170°C differieren.

Die Menge von II wird unabhängig von der Menge der Säure und der Menge von I zweckmäßigerweise so bemessen, daß sich II im stationären Betrieb vornehmlich im Mittelteil der Kolonne aufhält und in größeren Anteilen weder in den Sumpf noch in der Kopfbereich der Fraktionierkolonne gelangt. Enthalten Sumpf- und Kopffraktion dennoch größere Anteile an II, so sind zusätzliche Trennoperationen zweckmäßig oder erforderlich, also die Fraktionierungen von I und II einerseits und Ameisensäure, gegebenenfalls auch etwas Wasser, und II andererseits. Diese zusätzlichen Trennoperationen sind aber unproblematisch und beeinträchtigen die Wirtschaftlichkeit des Gesamtverfahrens der Säuregewinnung praktisch nicht.

Die Anzahl der theoretischen Trennstufen (Böden) hängt von der jeweiligen Destillationsaufgabe ab, also von der Art des Lösungsmittels I, und läßt sich daher nicht für alle Fälle allgemein angeben. In der Regel liegt diese Anzahl indes zwischen 5 und 50, meistens zwischen 15 und 25 Trennstufen.

Im allgemeinen nimmt man die Destillation unter vermindertem Druck vor, wobei der Druck gerade soweit gesenkt wird, daß Zersetzungsreaktionen infolge erhöhter Temperatur nicht mehr störend ins Gewicht fallen. Ein geringerer Druck als 40 mbar ist im allgemeinen nicht erforderlich.

Die Mitverwendung der Carbonsäureamide II gestattet eine deutliche Absenkung der Temperatur im mittleren Bereich der Kolonne. Die—um etwa 10—50°C—geringeren Temperaturen bewirken eine geringere thermische Belastung des Destillationsgutes oder sie ermöglichen das wirtschaftlichere Arbeiten bei relativ höherem Druck. Da außerdem die Trennleistung zunimmt und da deshalb zur Erzielung des gleichen Ergebnisses das Rücklaufverhältnis reduziert werden kann, verringert sich auch der Energiebedarf.

Beispiel

Gewinnung von weitgehend wasserfreier Ameisensäure aus einem Gemisch mit N-Di-n-butylformamid und etwas Wasser

Als Versuchsapparatur diente eine 1,3 m lange Füllkörperkolonne mit 4 cm Innendurchmesser. Die Kolonne war mit gläsernen Raschigringen (Durchmesser 3 mm) gefüllt, die eine theoretische Trennstufenzahl von etwa 21 Böden ergaben. Die Destillation wurde bei einem Kopfdruck von 80 mbar durchgeführt.

Die Kolonne wurde auf Höhe des 9. Bodens (von unten gezählt) über die Versuchsdauer von 96 Stunden kontinuierlich mit einem auf 45°C vorgewärmten Gemisch aus 65 g/h Ameisensäure, 2,5 g/h Wasser und 433,5 g/h N-Di-n-butylformamid beschickt, wobei sich am Kopf der Kolonne eine Temperatur von 35°C und am Sumpf von 155°C einstellte.

Nach Einstellung eines stationären Betriebszustandes wurden einmalig 30 g Monomethylformamid auf Höhe des 9. Bodens zugegeben. Bei einem Rücklaufverhältnis von 1,0 fielen pro Stunde 67,5 g 96-gew.%ige wäßrige Ameisensäure an, deren Stickstoffgehalt unter der Nachweisgrenze von 20 ppm lag.

Als Sumpfprodukt fielen 432,5 g/h N-Di-n-butylformamid an, in welchem keine Ameisensäure und auch kein Monomethylformamid mehr nachgewiesen werden konnte.

In einem Vergleichsversuch ohne Mitverwendung eines Carbonsäureamids II, mußte bie gleicher Sumpftemperatur (155°C) das Rücklaufverhältnis auf 1,5 erhöht werden. Trotz des hierdurch bedingten Mehrverbrauchs an Energie enthielt das Sumpfprodukt noch 0,5 Gew.-% Ameisensäure.

Dieses Ergebnis bestätigt, daß bei Mitverwendung geringer Mengen eines Carbonsäureamids II eine vollständige Rückgewinnung der Ameisensäure ermöglicht und gleichzeitig der Energiebedarf um etwa 20% verringert wird. Außerdem ist bei der Destillation mit einem Carbonsäureamid II keine Zersetzung der Ameisensäure zu beobachten.

**Patentansprüche**

1. Verfahren zur destillativen Gewinnung von Ameisensäure aus deren Gemischen mit Lösungsmitteln der allgemeinen Formel (I)

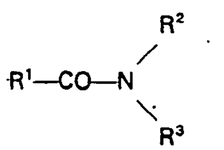

(I)

in der $R^1$ für Wasserstoff, die Methyl-, Ethyl- oder Vinylgruppe steht, und in der $R^2$ und $R^3$ Alkyl-, Cycloalkyl- Aryl- oder Aralkylgruppen oder gemeinsam eine 1,4- oder 1,5-Alkylengruppe mit jeweils 1 bis 8 C-Atomen mit der Maßgabe bedeuten, daß die Summe der C-Atome von $R^2$ und $R^3$ 7 bis 14 beträgt und daß nur einer dieser Reste eine Arylgruppe ist, dadurch gekennzeichnet, daß man die Destillation in Gegenwart eines Carbonsäureamids vornimmt, das tiefer als das Lösungsmittel I siedet.

2. Verfahren nach Anspruch 1, dadurch gekennzeichnet, daß das Lösungsmittel (I) N-Di-n-butylformamid ist.

3. Verfahren nach den Ansprüchen 1 und 2, dadurch gekennzeichnet, daß man als Carbonsäureamid ein N-Methyl-carbonsäureamid verwendet.

4. Verfahren nach den Ansprüchen 1 und 2, dadurch gekennzeichnet, daß man als Carbonsäureamid ein N,N-Dimethylcarbonsäureamid verwendet.

**Revendications**

1. Procédé pour la récupération de l'acide formique par distillation à partir de mélanges de celui-ci avec des solvants de formule générale (I)

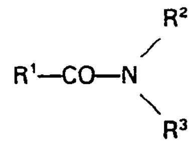

(I)

dans laquelle $R^1$ est mis pour un atome d'hydrogène, le groupe méthyle, éthyle ou vinyle et dans laquelle $R^2$ et $R^3$ représentent des groupes alkyle, cycloalkyle, aryle ou aralkyle ou forment en commun un groupe 1,4- ou 1,5-alkylène à 1—8 atomes de C dans chaque cas, avec cette condition que la somme des atomes de C de $R^2$ et $R^3$ soit comprise entre 7 et 14 et qu'un seul de ces radicaux soit un groupe aryle, caractérisé en ce qu'on procède à la distillation en présence d'un amide d'acide carboxylique dont le point d'ébullition est inférieur à celui du solvant I.

2. Procédé selon la revendication 1, caractérisé en ce que le solvant (I) est le N-di-n-butylformamide.

3. Procédé selon la revendication 1 ou 2, caractérisé en ce qu'on utilise comme amide d'acide carboxylique un amide d'acide N-méthyl-carboxylique.

4. Procédé selon la revendication 1 ou 2, caractérisé en ce qu'on utilise comme amide d'acide carboxylique un amide d'acide N,N-diméthylcarboxylique.

**Claims**

1. A process for the recovery of formic acid, by distillation, from its mixture with a solvent of the general formula (I)

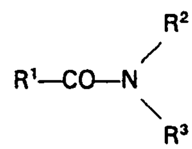

(I),

where $R^1$ is hydrogen, methyl, ethyl or vinyl, and $R^2$ and $R^3$ are each alkyl, cycloalkyl, aryl or aralkyl, or $R^2$ and $R^3$ together form a 1,4- or 1,5-alkylene group of 1 to 8 carbon atoms, with the provisos that the sum of the number of carbon atoms in $R^2$ and $R^3$ is 7 to 14 and that only one of these radicals is aryl, wherein the distillation is carried out in the presence of a carboxamide which has a boiling point lower than that of the solvent I.

2. A process as claimed in claim 1, wherein the solvent I is N-di-n-butylformamide.

3. A process as claimed in claims 1 and 2, wherein an N-methylcarboxamide is used as the carboxamide.

4. A process as claimed in claims 1 and 2, wherein an N,N-dimethylcarboxamide is used as the carboxamide.